# EUROPEAN PATENT APPLICATION

(11) **EP 4 541 365 A1**
(43) Date of publication of application: **23.04.2025**
(21) Application number: 23204384.4
(22) Date of filing: 18.10.2023
(51) Int. Cl.: A61K 38/09, A61K 9/19, A61P 35/00

(54) **TEVERELIX-TFA IN A DOSAGE REGIME FOR USE IN THE TREATMENT OF PROSTATE CANCER**

(71) Applicant: Antev Limited, Weybridge KT13 8AH (GB)
(72) Inventor: MacLean, Carol, London, W1S 1DN (GB); Van Os, Steve, London, W1S 1DN (GB)
(74) Representative: Holme Patent A/S

(57) **Abstract**

The present invention relates to a dosage regime for a teverelix-TFA composition used for treating prostate cancer. Said dosage regime comprises a loading dose of 360 mg SC and 180 mg IM, with a low maintenance dose of the teverelix-TFA composition (360 mg SC) every six weeks starting at day 28. Thus, the dosage regime according to the invention provides an improve dosage regimens in which a castration rate of ≥95% is obtained in a treatment period of 52 weeks, and with fewer side effects and adverse events, thereby providing an optimised response/side effect relationship.

## Description

The present invention relates to a composition comprising teverelix-TFA for the use in the treatment of prostate cancer and to a dosage regime for use in said treatment.

Prostate cancer is the second most common cancer in men worldwide, accounting for 15% of cancers diagnosed in men. The choice of treatment depends on a number of factors, e.g. the rate at which the cancer is growing, whether metastasis has set in, and most importantly implicated benefits and side effects of the treatment methods to be undertaken.

Prostate cancer relies on the androgenic hormones that mainly are produces in the testicles to proliferate, and treatment of advanced and/or metastasized prostate cancer therefore often involves androgen deprivation therapy (ADT). This is often achieved using bilateral orchiectomy or medical castration via administration of luteinizing hormone-releasing hormone (LHRH) analogs or antagonists both of which effectively suppresses testosterone production.

Teverelix (Ac-D-Nal-D-pClPhe-D-Pal-Ser-Tyr-D-Hci-Leu-Lys(iPr)-Pro-D-Ala-NH₂) is a synthetic gonadotropin-releasing hormone antagonist (GnRH antagonist) and will accordingly compete with the endogenous neurohormone GnRH (also known as luteinizing hormone releasing hormone, LHRH) for binding to its receptors in the anterior pituitary gland. By decreasing or blocking GnRH action, teverelix suppress release from the anterior pituitary gland of follicle stimulating hormone (FSH) and luteinizing hormone (LH).

In males, FSH plays an important role in spermatogenesis and LH stimulates production of testosterone in the testes. Accordingly teverelix can be used in an androgen deprivation therapy for the treatment of prostate cancer.

Despite the fact that considerable success has been achieved in the treatment and management of prostate cancer using androgen deprivation therapy, such therapy has, however, been associated with acute and long-term side effect and/or adverse events, such as hyperlipidemia, fatigue, hot flashes, flare effect, osteoporosis, insulin resistance, cardiovascular disease, anaemia, and sexual dysfunction [Seidenfeld et al. Single-Therapy Androgen Suppression in Men with Advanced Prostate Cancer: A Systematic Review and Meta-Analysis. Ann. Intern. Med. 2000;132:566-577].

Especially cardiovascular diseases have proven to be of significant importance in this respect, since cardiovascular diseases today are the leading cause of death in men with prostate cancer.

A recent meta-analysis, see Li Gu et al., Adverse cardiovascular effect following gonadotropin-releasing hormone antagonist versus GnRH agonist for prostate cancer treatment: A systematic review and meta-analysis, Front. Endocrinol., 31 March 2023 Sec. Cancer Endocrinology Volume 14 - 2023, concluded that prostate cancer patients treated with GnRH antagonists are associated with lower occurrences of cardiovascular events, cardiovascular death and myocardial infarction than those treated with GnRH agonists.

It is accordingly preferred to use GnRH antagonists (such as teverelix) in androgen deprivation therapy, but special considerations must be taken to ensure that any undesirable adverse effects, e.g. cardiovascular diseases, are reduced.

Several different dosage regimes using relatively low doses of teverelix are known for treatment of prostate cancer. It is further known to administer teverelix intramuscularly, subcutaneously and via a combined subcutaneously/intramuscularly administration; see e.g. the applicant's international application WO2018138703.

However, even though the dosage regimes disclosed in WO2018138703 provides beneficial results for some patients, a considerable amount of patients are either not castrated at all or do not remain castrated throughout the entire treatment period. Accordingly the dosage regimes disclosed in WO2018138703 have proven to be less effective during the relatively long treatment period of prostate cancer patients.

Thus, there remains a demand for an optimised dosage regime for treatment of prostate cancer, which dosage regime provides an optimised dose-response relationship in which relatively low doses of teverelix can be used to reduce the side effect (adverse events) associated with GnRH antagonists, and at the same time ensures that patients remain castrated during the entire treatment period.

It is accordingly a first aspect of the present invention to provide a dosage regime for a teverelix-TFA composition for the treatment of prostate cancer, especially advanced prostate cancer, which dosage regimes provides fewer side/adverse effects than e.g. treatment with the conventional GnRH agonists and GnRH antagonists.

It is a second aspect of the present invention to provide a dosage regime for a teverelix-TFA composition, wherein said dosage regime prolongs the circulatory half-life of teverelix, thereby prolonging the duration of the therapeutic effect of teverelix *in vivo.*

It is a third aspect of the present invention to provide a dosage regime for a teverelix-TFA composition in which at least 95% of all patients receiving the treatment is clinical castrated (testosterone <0.5 ng/mL) within a few days, and remains clinical castrated during the entire treatment period.

It is a fourth aspect of the present invention to provide a dosage regime in which the patient can receive the required medication during a single visit at e.g. a medical facility, instead of having to have individual injections on several consecutive days.

These and further aspects are achieved according to the present invention by providing a teverelix-TFA composition for use in the treatment of prostate cancer, wherein said composition is administered via a dosage regime comprising a loading dose and at least one maintenance dose, and wherein the loading dose comprises
- administering 360 mg of the teverelix-TFA composition subcutaneously,
- administering 180 mg of the teverelix-TFA composition intramuscularly, and
wherein the subcutaneously and intramuscularly administration are carried out substantially simultaneously.

The combined administration procedure of the loading dose, i.e. the subcutaneously (SC) and intramuscularly (IM) administration has proven to improve the pharmacokinetic/pharmacodynamic (PK/PD) profile of teverelix.

Even though the differences between PK profiles following subcutaneous or intramuscular or combined subcutaneous/intramuscularly loading dose scenarios become normalized over a period, the inventors have found that the PK profile in first four weeks is important for maintaining a desired castration rate during a treatment period of at least 26 weeks, preferably at least 52 weeks.

The inventors of the present invention have in this respect found that in order to achieve immediate castration and maintain said castration for the first four weeks, a teverelix-TFA composition loading dose of 360 mg SC and 180 mg IM has proven to be important in maintaining a castration rate of ≥95% in a patients during the entire treatment period.

Loading with other doses e.g. a 360 mg SC dose alone, a 360 mg IM loading dose alone, or lower combined loading doses of a teverelix-TFA with a combined SC+IM administration, as e.g. disclosed in WO2018138703, has proven not to sustain the teverelix plasma concentrations required to achieve the desired castration rate of ≥95% for the entire treatment period.

Testosterone level ≤ 0.5 ng/ml has been used to define castrate level after surgery or after androgen deprivation treatment (ADT) in metastatic prostate cancer. The inventors of the present invention has shown that the dosage regime according to the invention is capable of providing a blood plasma testosterone concentration that maintains castration levels, i.e. less than about 0.5 ng/mL, determined by a liquid chromatography/mass spectrometry (LC-MS) assay, and maintaining said concentration below about 0.5 ng/mL during the entire treatment period of at least 52 weeks for at least 95 % of all patients, i.e. with a castration rate of ≥95%.

The inventors have further shown that the dual administration with a loading dose of 360 mg SC + 180 mg IM (540 mg in total) prevents the formation of a "dead"-phase in which insufficient teverelix is released. The faster rate absorption of the IM administration as loading dose, offsets the slower second peak observed from the SC loading dose, whereby the combined dual administration ensures sufficient plasma teverelix levels to both achieve and maintain the desired castration rate of ≥95%. Consequently the teverelix-TFA composition when administered via the dual administration process in the high loading doses according to the invention provides a continuous, more uniform and appropriate release of teverelix than hitherto known.

Thus, the dosage regime according to the invention will produce a surprising and unexpected prolonging of the circulatory half-life of teverelix both relative to the individual injections alone, and relative to lower loading doses, thereby obtaining faster and more efficient castrations, as well as prolonging the duration of a therapeutic effect of teverelix *in vivo.*

The different administrations routes of the loading dose can be performed in any suitable order. For example, the intramuscularly administration be performed before the subcutaneously administering, or the administration order may be reversed, i.e. the teverelix-TFA composition is administered subcutaneously before the intramuscularly administration. It is however relevant that the subcutaneously and intramuscularly administrations are performed substantially simultaneously in order to obtain the desired effect.

Within the context of the present invention the term "simultaneously" refers to a time period that is practically possible for the person performing the administrations and it is preferred that the teverelix-TFA composition is administered within a period of not more than 1 hour. It is however preferred that the loading dose of 180 mg teverelix-TFA intramuscularly and 360 mg teverelix-TFA subcutaneously is administered within a period of not more than 10 minutes and even more preferred within a period of not more than 5 minutes, e.g. around 1 - 2 minutes.

The intramuscularly and subcutaneously administrations are preferably performed via injection e.g. with a conventional syringe or a jet-injector.

In order to ensure that the patient remains medically castrated during the required treatment period, one or more maintenance doses are preferably administered at predefined intervals after the loading dose.

Said maintenance dose is in a preferred embodiment 360 mg of the teverelix-TFA composition and said maintenance dose is preferably administered subcutaneously. Even though higher maintenance doses of the teverelix-TFA composition may also be capable of maintaining the desired castration rate of ≥95%, the inventors of the present invention have found that 360 mg administered subcutaneously (360 mg SC) is the lowest possible maintenance dose that can provide the desired castration rate of ≥95% during a treatment period of 52 weeks.

Thus, using a maintenance dose of the teverelix-TFA composition 360 mg SC, i.e. a relatively low dose, will effectively reduce the adverse events and side effects, and at the same time ensure the desired medical castration. Accordingly, said maintenance dosage provides that right balance of benefits in relation to both to costs and adverse events.

The first maintenance dose is preferably administered on day 28 after the loading dose (SC + IM), and may be followed by a second and, if relevant, further maintenance doses of 360 mg of the teverelix-TFA composition. All maintenance doses are preferably administered subcutaneously, as this has proven to provide the best results.

The duration between the second and optionally further maintenance doses, calculated from the first maintenance dose, is preferably six weeks. Thus, the second and further maintenance doses follow a six-weekly (Q6W) dosing schedule.

Longer duration between the maintenance doses, e.g. eight-weeks has proven not to be able to maintain the desired castration rate of ≥95% unless the patient receive at least twice the dose of the teverelix-TFA composition. Thus, in order to reduce and minimise any dose-dependent side effect and/or adverse-events, in particular the cardiovascular diseases associated with GnRH-antagonists, it is relevant to use the lowest possible dose of teverelix while the castration rates are maintained during the treatment period.

Thus, using the dosage regime according to the invention in which the teverelix-TFA composition is administered with a loading dose of 360 mg SC and 180 mg IM, the inventors have shown that a only a low maintenance dose of the teverelix-TFA composition, 360 mg SC is required, and that said maintenance dose only needs to be administered at relatively long intervals, every six weeks starting at day 28. Thus, the dosage regime according to the invention provides an improve dosage regimens compared to the know dosage regimens.

Furthermore, the dosage regime according to the present invention improves patient compliance, as said patents can receive the loading dose during a single visit at e.g. a medical facility, instead of having to receive several (e.g. three) subcutaneous injections on three consecutive days in order to obtain sufficient serum concentrations for achieving immediate castration, preferably within 72 hours after the loading dose is administered.

A dosage regime of the teverelix-TFA composition according to the invention, i.e. with a loading doses of 360 mg SC + 180 mg IM followed by a maintenance dose of 360 mg SC Q6W starting at day 28 has proven to be the lowest possible doses that is capable of achieving the desired ≥ 95% castration rate in treated human subject in up to 52 weeks.

For the teverelix to be absorbed in the body, said teverelix must preferably be present in the form of solution at the site of absorption. Various techniques are used for the enhancement of the solubility of poorly soluble drugs, such as teverelix, however it has been found that crystal engineering, in which the teverelix and trifluoroacetate (TFA) provides an organisation of the teverelix peptide and its counter-ion TFA in a crystalline structure provides an effective sustained release of teverelix, having improve physicochemical properties (e.g., solubility and stability), and improve efficacy (e.g., bioavailability).

However, Teverelix (Ac-D-Nal-D-pClPhe-D-Pal-Ser-Tyr-D-Hci-Leu-Lys(iPr)-Pro-D-Ala-NH₂) has a tendency of forming gels in the presence of counter ions. Thus, in order to obtain the desired microcrystalline suspension without any undesirable gel-formation, the applicant has proven see e.g. WO2020007852 and WO2020007857 that when the molar ratio of teverelix to the counter-ion trifluoroacetate (TFA) is at least 1:2, preferably at least 1:2.2 and preferably not above 1:2.8 a stable, homogenous microcrystalline suspension is provided that does not contain any gel. Furthermore such suspensions will comprise both soluble and insoluble teverelix, thereby providing a unique bioavailability of teverelix.

Different methods to obtain and adjust said molar ratios to the desired molar ratios according to the invention, are disclosed in the applicant's applications WO2020007852 and WO2020007860.

Within the content of the present invention the term "molar ratio of teverelix to trifluoroacetate" refers to the molar relationship between teverelix and trifluoroacetate, where the first number of the molar ratio is the mol content of teverelix in the composition and the second number refers to the mol content of TFA in the composition. For instance, a molar ratio of 1:2.2 means that for each mol teverelix in the composition, said composition comprises 2.2 mol TFA, and a molar ratio of at least 1:2.2 means that for each mole teverelix in the composition, the composition comprises at least 2.2 mol trifluoroacetate (TFA).

Without being bound by theory, the soluble teverelix is in the form of an aqueous solution, and in some situations a gel. The presence of a gel will inhibit any freely aqueous teverelix and therefore prevent, or at least reduce, immediate release. The insoluble teverelix is in the form of microcrystals. Said microcrystals will prevent gel formation, therefore "unlocking" the aqueous teverelix. Over time the TFA in the composition according to the invention will be absorbed by the body, lowering the ratio, so the microcrystals subsequently turn in to gel, which forms the slow release depot. Thus, the non-gel-soluble teverelix is immediately available, providing an almost immediate onset of action, and the gel-soluble and insoluble teverelix (microcrystals) will assist in providing a sustained release of teverelix. Accordingly the composition according to the invention provides a soluble-insoluble transition at the administration site, and accordingly a sustained release of teverelix.

Thus, the teverelix-TFA composition used in the dosage regime according to the invention has both an immediate onset of action leading to a fast suppression of the gonadotropins, but also a sustain release of teverelix thereby ensuring that the subject maintains a therapeutically effective concentration in the blood plasma at least until a maintenance dose is administered. This will not only provide a more reliable composition for the treatment of prostate cancer, but also improve patient compliances as fewer administrations (e.g. injections) are required. It is in this respect preferred that the patient can be treated in a single visit, i.e. that said patient does not have to visit a medicinal facilities several consecutive days.

This dual release mode of the teverelix-TFA composition is enhanced by the combined administration (SC+IM) of the loading dose, in which the faster rate absorption of the intramuscular administration offsets the slower second release peak observed from the subcutaneously administration. Thereby will the dosage regime according to the invention both provide a fast and efficient castration of the patient, but also a sustained and controlled release of teverelix over time thereby ensuring that the patient remains medically castrated during the treatment period, or at least until the first maintenance dose is administered. Thus, the dosage regime according to the invention can both be used in the treatment of patients with prostate cancer where fast control of disease is needed and for patients where only sustained release is relevant.

As an example of the benefits of the dual administration of the composition according to the invention the prostate size may be reduced in the subject after having received the loading dose and one or more maintenance doses. Similarly, the urine flow may be increased in the subject during the treatment period.

Even though the patient may be any kind of primate, it is preferred that the patient is a male human.

The dosage regime is intended for treating prostate case, especially advanced prostate cancer; however the dosage regime may also be used for treating other conditions in which a lower testosterone level is required. Elevated testosterone levels can also be a result of hyperthyroidism, Grave's disease, precocious puberty, or other kinds of cancer. Similar the teverelix-TFA composition can be used as a means for male birth control.

The dosage regime according to the invention will ensure that the plasma teverelix concentration is sufficient to obtain a medical castration of the subject. Thereafter the teverelix-TFA composition will have a sustained release of teverelix maintaining the plasma teverelix concentration in order to ensure that the subject will remain castrated until the administration of a maintenance dose.

The dosage regime will in addition to reducing the blood plasma testosterone concentration preferably also reduce the subject's blood plasma prostate specific antigen (PSA) concentration to less than about 4 ng/mL. PSA is a substance made by cells in the prostate gland (both normal cells and cancer cells). PSA is mostly found in semen, but a small amount is also found in the blood. Incidence of an enlargement of a prostate, such as through a condition such as BPH or prostate cancer, can increase an amount of PSA in a subject. Therefore, PSA levels can be used as a metric for treatment of a condition resulting in the increase in PSA levels.

The invention also relates to embodiments in which an additional pharmaceutical substance is administered. Said additional pharmaceutical substance can e.g. be co-administered together with the loading dose of teverelix-TFA, with the subsequent maintainance dose of teverelix-TFA, or independently of the administration of teverelix-TFA, e.g. before, between or after administration of the respective loading dose and maintainance dose(s).

The additional pharmaceutical substance may be any substance capable of providing a beneficial effect on the condition or disease to be treated by the composition according to the invention. As an example can be mentioned that the additional pharmaceutical substance may be an antineoplastics, adrenergic receptor antagonists and/or 5α-reductase inhibitors.

The present invention also relates to a pharmaceutical composition comprising the teverelix-TFA composition according to the invention, and in which the pharmaceutical composition further comprises a pharmaceutically acceptable excipient, diluent, or carrier.

The diluent may in a simple embodiment be water but can also be an aqueous buffering agent, e.g. saline, citrate, phosphate, acetate, glycine, tris(hydroxymethyl)aminomethane (tris), 4-(2-hydroxyethyl)-1-piperazineethanesulfonic acid (HEPES) and/or piperazine-N,N'-bis(2-ethanesulfonic acid) (PIPES).

In some embodiments, the formulation further comprises an isotonic agent, e.g. a polyhedric alcohol, a sugar alcohol and/or mannitol.

The present invention also relates to a kit comprising the teverelix-TFA composition. The kit may further comprise means to perform a duel injection as described herein, as well as instructions for performing said dual injection. In some aspects, the teverelix-TFA composition or formulation can be packaged in a container e.g. a vial, and the kit can further comprise at least one syringe and needle for administration of said composition. In some exemplary embodiments, a pair of needles can be provided of proper length for injection via intramuscular and subcutaneous injection. Instructions for such an administration can be packaged in the kit.

In some embodiments, the teverelix-TFA composition or formulation can be provided as a lyophilized powder contained in a vial, e.g. obtained via the method described in WO2020007860. A kit can comprise a vial of sterile water or TFA-solution for reconstitution/resuspension of the lyophilized teverelix, as well as instructions for preparation. In other instances, a syringe comprising an integrated water chamber can be provided to re-suspend the lyophilized teverelix-TFA composition or formulation within the syringe prior to administration.

A better understanding of present invention will be will be obtained by reference to the following discussion of the provided experimental results.

### Experiments

In order to deprive an optimal dosage regime of teverelix-TFA that can be used in future evaluation in phase 3 clinical trails, a Model Informed Drug Development (MIDD) approach was used to obtain a pharmacokinetic and pharmacodynamic analysis of teverelix in order to provide a population PK-PD model, which described the concentration profile accommodating both IM and SC routes of administration.

The MIDD approach use a variety of quantitative methods to help balance the risks and benefits of drug products in development. When successfully applied, MIDD approaches can improve clinical trial efficiency, increase the probability of regulatory success, and optimize drug dosing/therapeutic individualization in the absence of dedicated trials

The population PK-PD model for Teverelix-TFA was based on concentration and efficacy data obtained from 273 patients from nine phase 2 clinical studies.

An overview of the clinical phase 2 studies included in the development of the population PK-PD model, is shown in table 1 below.

**Table 1 Clinical phase 2 studies included in the development of the population PK-PD model**

| **Study number/ EudraCT** | **Study location(s)** | **Study period** | **Indication** | **Design** | **Treatment regimen** | **No of subjects receiving teverelix/ placebo** |
|---|---|---|---|---|---|---|
| ARD-0301-001 | Clinic of Urology, MOKB, Minsk, Belarus | Oct 2005 - June 2006 | Benign prostatic hyperplasia | Randomized, placebo-controlled, | Single dose of 30 mg SC or 60 mg SC | |
| | 4^{th} City Hospital, Minsk, Belarus | | | | | 28/14 |
| | 9^{th} City Hospital, Minsk, Belarus | | | | | 28/14 |
| | City Hospital of Emergency, Minsk, Belarus | | | | | |
| ARD-0301-002* | Clinic of Urology, MOKB, Minsk, Belarus | Oct 2005 - Oct 2006 | Benign prostatic hyperplasia | Randomized, placebo-controlled, double-blind | Single dose of 30 mg SC or 60 mg SC | |
| | 4^{th} City Hospital, Minsk, Belarus. | | | | | 25/26* |
| | 9^{th} City Hospital, Minsk, Belarus. | | | | | 26* |
| | City Hospital of Emergency, Minsk, Belarus | | | | | |
| ARD-0301-003 / 2005-002100-42 | Oncology Institute of Vilnius University, Vilnius, Lithuania | Oct 2005 - Dec 2005 | Advanced prostate cancer | Single centre, non-controlled, open-label | 2 doses of 120 mg SC, interval 24 h | 8/NA |
| ARD-0301-008 / 2005-005742-39 | Oncology Institute of Vilnius University, Vilnius, Lithuania | Feb 2006 - Jul 2006 | Advanced prostate cancer | Single centre, non-controlled, open-label | 2 doses of 120 mg SC, interval 24 h | 8/NA |
| ARD-0301-010 / 2006-004572-13 | Oncology Institute of Vilnius University, Vilnius, Lithuania | Nov 2006 - Jul 2007 | Advanced prostate cancer | Multicentre, non-controlled, open-label | 3 doses of 120 mg SC or 180 mg SC, interval 24 h | 18/NA |
| | LSMU Kaunas Hospital, Kaunas, Lithuania | | | | | 20/NA |
| | P. Stradins Clinical University Hospital, Riga, Latvia | | | | | |
| | Latvian Centre of Oncology, Riga, Latvia | | | | | |
| | Liepaja Reginal Hospital, Liepaja, Latvia | | | | | |
| | Lithuania and Latvia(2 centers in each country) | | | | | |
| EP-24332T-A012 | Clinic of Urology, MOKB, Minsk, Belarus | | Benign prostatic hyperplasia | Randomized, placebo-controlled, double-blind | 2 doses of 60 mg SC, interval 48 h | |
| | 4th City Clinical Hospital, Minsk, Belarus | | | | | 41/40 |
| | 19th City Hospital, Minsk, Belarus | | | | | |
| | City Hospital of Emergency Minsk Russia | | | | | |
| | St John Emergency Hospital, Bucharest, Romania | | | | | |
| | University Hospital Alexandrovska, Sofia, Bulgaria | | | | | |
| | Klaipeda University Hospital, Klaipeda, Lithuania | | | | | |
| | National Cancer Institute, Vilnius, Lithuania | | | | | |
| EP-24332T-A013 | Vilnius University Hospital, Vilnius, Lithuania LSMU Kaunas Hospital, Kaunas, Lithuania | May 2004 - Sep 2004 | Advanced prostate cancer | Multicentre, non-controlled, open-label | 3 doses of 90 mg SC, interval 24 h | 14/NA |
| EP-24332T-A014 / 2004-001648-64 | Vilnius University Hospital, Vilnius , Lithuania LSMU Kaunas Hospital, Kaunas, Lithuania | Oct 2004 - March 2005 | Advanced prostate cancer | Multicentre, non-controlled, open-label | 2 doses of 90 mg IM, interval 1 week | 14/NA |
| ANT-1111-02 / | Vilnius University Hospital, Vilnius, Lithuania | Jan 2021 - | Advanced | Multicentre, | Group 1: | |
| 2020-000543-31 | LSMU Kaunas Hospital, Kaunas, Lithuania Klaipeda | Feb 2023 | prostate cancer | non-controlled, | LD: 120 mg IM + 120 mg SC | 9/NA |
| | University Hospital, Klaipeda, Lithuania | | | open-label | MD : 4 doses of 120 mg SC | |
| | National Cancer Institute, Vilnius, Lithuania | | | | Q6W | |
| | | | | | Group 2: | |
| | | | | | LD: 180 mg IM + 180 mg SC | |
| | | | | | MD: 4 doses of 180 mg SC | 41/NA |
| | | | | | Q6W | |

| | | | | | | |
|---|---|---|---|---|---|---|
| SC: subcutaneous; IM: intramuscular; LD: Loading dose MD: maintenance dose; NA: not applicable * Study ARD-0301-002 was a follow-on of Study ARD-0301-001. 51 of the 56 subjects on active treatment from Study ARD-0301-001 continued with active treatment in Study ARD-0301-002. For PK purposes these subjects were considered unique because subject identities in the two studies would not be linked. | | | | | | |

Data from a total of nine phase 2 clinical studies that included 273 male BPH and advanced prostate cancer (APC) patients treated with single or multiple dose regimens (ranging from 30 mg - 360 mg per day) were utilized in the current analysis.

For PK modelling six patients (four patients from study ARD-0301-010, one patient from study EP-24332T-A012 and one patient from study ARD-0301-008) were excluded due to numerical difficulties in model convergence. One additional patient (from study EP-24332T-A012) was excluded from pharmacodynamic modelling due to numerical difficulties in model convergence.

A summary of demographic data of patients included in the population PK-PD model is provided in Table 2.

**Table 2 Demographic summary of subjects included in the PK-PD model. 51 of the 56 subjects on active treatment from Study ARD-0301-001 continued with active treatment in Study ARD-0301-002. For PK purposes these subjects were considered unique and subject identities in the two studies would not be linked.**

| **Parameter** | **Summary** |
|---|---|
| **Age (year)** - Mean (min-max) | 68.3 (23-84) |
| **Weight (Kg)** - Mean (min-max) | 82.1 (59-120) |
| **Race** - White (n [%]) | 273 (100%) |
| **Sex -** Male (n [%]) | 283 (100%) |
| **Creatinine clearance (uM)** - Mean (min-max) | 91.7 (50-162) |

Participants in the studies received single or multiple doses of Teverelix-TFA as SC and/or IM injections.

Teverelix-TFA was supplied as a freeze-dried powder without excipient, which was reconstituted just prior to injection with 5% mannitol (w/v) in water for injection to a final concentration of 75 mg/mL. The study medication was administered as SC injections into the lower abdominal wall and/or as IM injections into the buttock.

SC injections were administered at five dose levels: 30 mg, 60 mg, 90 mg, 120 mg, and 180 mg. IM injections were administered at three dose levels: 90 mg, 120 mg, and 180 mg. Treatment regimen for each study are shown in Table 1.

Serial blood samples for PK evaluation were taken after administration of Teverelix-TFA. The blood sampling schedules differed between studies and depended on the number of drug administrations, see Table 3 below.

In the trials with APC patients, dense sampling was used in the first 24 hours after drug administration, followed by daily and then weekly sampling. This was followed by less frequent sampling until the effect of the treatment had ceased, i.e. until 2 consecutive, increasing testosterone levels above castration level were recorded with the latter one above 2 ng/mL.

In the trials with BPH patients less dense sampling was used, see Table 3. Plasma teverelix concentrations were determined by liquid chromatography with tandem mass spectrometry at HFL Ltd, Fordham, UK (now part of U.S. Alliance Pharma) for studies ARD-0301-001 and ARD-0301-002 and by radio-immunoassay (RIA) at Professor Huy Ong's laboratory at the University of Montreal, Canada for all other studies.

Blood samples for PD (testosterone) evaluation were taken less frequently compared to PK samples, with sampling schedules appropriate for the study design. In general PD sampling started with daily assessments, followed by weekly assessments. Sampling continued with a lower frequency, depending on study duration and study design, see Table 3, until the effect of the treatment had ceased.

Serum concentrations of testosterone were analyzed by the central laboratory MDS Pharma Services, Hamburg, Germany using a commercially available RIA kit (MD & Biochemicals INC), except for study ANT-111-02, for which samples were analyzed via LC-MS/MS at SGS laboratory in Munich.

Blood sampling for testosterone was done along with blood sampling for PK, to enable PK-PD modelling.

**Table 3 Summary of PK and PD data utilized in the development of the population PK-PD model**

| **Study number** | **No. of subjects included in the PK-PD analysis** | **Parameters included in the PK-PD model** | **Timing of PK and PD parameters** |
|---|---|---|---|
| ARD-0301-001 | 56 | Demographic data | PK: pre-dose, at 1, 2 and 4 h and at 1, 2 and 4 weeks post-dose |
| | | Plasma teverelix concentrations | PD: pre-dose and at 1,2 and 4 weeks post-dose |
| | | Serum T levels | |
| ARD-0301-002 (Follow-on study | 51 | Demographic data | PK: pre-dose, at 1, 2 and 4 h and at 1 and 2 weeks post-dose; and then 4-weekly until final assessment at Week 28 |
| | | Plasma teverelix concentrations | PD: pre-dose and at 1 and 2 weeks post-dose; and then 4-weekly until final assessment at Week 28 |
| ARD-0301-001) | | Serum T levels | |
| ARD-0301-003 | 8 | Demographic data | PK: pre-dose and at 1, 2, 4, 6, 10 and 24 h post-dose on Days 1 and 2; 48, 72 and 144h post-dose on Day 2; and then weekly until treatment has ceased to be effective* |
| | | Plasma teverelix concentrations | |
| | | Serum T levels | PD: pre-dose on Days 1 and 2; 24, 48, 72 and 144 post-dose on Day 2; and then weekly until treatment has ceased to be effective* |
| ARD-0301-008 | 8^{#} | Demographic data | PK: pre-dose and at 1, 2, 4, 6, 10 and 24 h post-dose on Days 1 and 2; 48, 72 and 144h post-dose on Day 2; and then weekly until treatment has ceased to be effective* |
| | | Plasma teverelix concentrations | |
| | | Serum T levels | PD: pre-dose on Days 1 and 2; on 24, 48, 72 and 144 post-dose on Day 2; and then weekly until treatment has ceased to be effective* |
| ARD-0301-010 | 38^{#} | Demographic data | PK: pre-dose and at 1, 2, 4, 6, 10 and 24 h post-dose on days 1, 2 and 3; 48 and 144 h post-dose on Day 3; and then weekly until treatment has ceased to be effective* |
| | | Plasma teverelix concentrations | |
| | | Serum T levels | PD: pre-dose on Days 1, 2 and 3; 24, 48 and 120 h post-dose on Day 3; and then weekly until treatment has ceased to be effective* |
| EP-24332T-A012 | 41^{#} | Demographic data | PK: pre-dose on Days 1 and Day 3; at the end of Weeks 1, 2, 3, 4, 8, 12 and at the final assessment (Week 16) |
| | | Plasma teverelix concentrations | PD: pre-dose on Days 1 and Day 3; at the end of Weeks 1, 2, 3, 4, 8, 12 and at the final assessment (Week 16) |
| | | Serum T levels | |
| EP-24332T-A013 | 14 | Demographic data | PK: pre-dose and at 1, 2, 4, 6, 10, post-dose Day 1, pre-dose Days 2 and 3 and 48 and 120 h post-dose day 3; and then weekly until treatment has ceased to be effective* |
| | | Plasma teverelix concentrations | |
| | | Serum T levels | PD: pre-dose on Days 1, 2 and 3 and 48 and 120 h post-dose on Day 3; and then weekly until treatment has ceased to be effective* |
| EP-24332T-A014 | 14 | Demographic data | PK: pre-dose and 1, 2, 4, 6, 10, 24, 48 and 96 h post-dose on days 1 and 8; and then weekly until treatment has ceased to be effective* |
| | | Plasma teverelix concentrations | |
| | | Serum T levels | PD: pre-dose and 24, 48 and 96 h post -dose on Days 1 and 8; and then weekly until treatment has ceased to be effective* |
| ANT-1111-02 | 50 | Demographic data | PK: pre-dose and 1, 1.5, 2, 2.5, 3, 4, 8, 12, 18, 24, 24, 48, 72 and 114 h and 7, 10, 12, 14 and 21, 28 and 35 days after the loading dose; prior to every 6-weekly maintenance dose until Week 24 and at the follow-up visit (Week 29). |
| | | Plasma teverelix concentrations | |
| | | Serum T levels | PD: pre-dose and 1, 2, 3, 4, 7, 10, 12, 14, 21, 28 and 35 days after the loading dose; prior to every 6-weekly maintenance dose until Week 24 and at the follow-up visit (Week 29) |

| | | | |
|---|---|---|---|
| PD: pharmacodynamics; PK: pharmacokinetics; T: testosterone * sampling continued until 2 consecutive, increasing T levels above castration level were recorded with the latter one above 2 ng/mL ^{#} one patient from study ARD-0301-008, four patients from study ARD-0301-010 and one patient from study EP-24332T-A012 and were excluded from the PK modelling ^{$} one patient from study EP-24332T-A012 was excluded from the PD modelling | | | |

A total of 3986 teverelix PK samples from 274 patients and 2964 testosterone PD samples from 273 patients were included in the PK-PD modelling. Pre-dose samples of teverelix before first dose and other samples below limit of quantitation and were excluded from the PK-PD modelling.

The clinical data were utilized in order to provide a model for Develop a population pharmacokinetic and pharmacodynamic (PK-PD) model to characterize plasma concentration profiles of teverelix (PK) and testosterone (T) in order to:
- conduct simulations using the PK-PD model to derive optimal loading and maintenance dosage regimens,
- to achieve clinical castration (testosterone <0.5 ng/mL by day 28), and
- to sustain clinical castration for 26 weeks, and preferably 58 weeks after the onset.

### Population PK-model

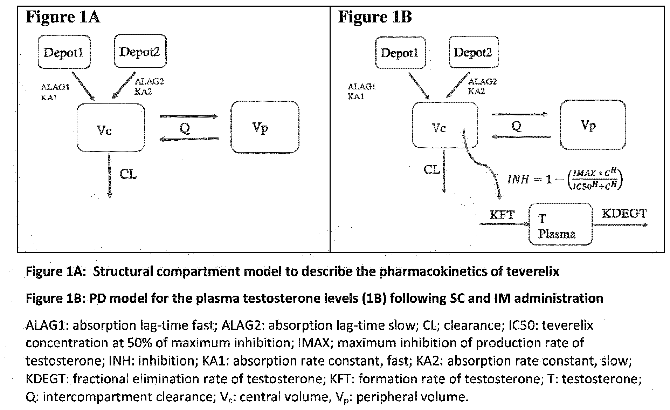

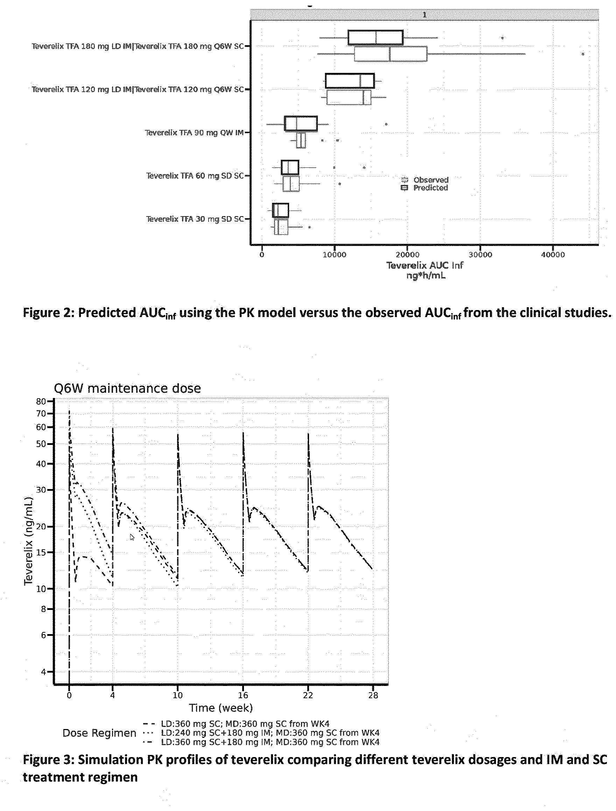

A two-compartment model with a two sequential first order absorption process (fast and slow absorption components) was selected as structural model to describe the PK of teverelix after SC and IM administration, see Figure 1A. Separate absorption rate constants and absorption lag times for IM and SC routes of administration were modelled. No further covariates needed to be included in the model, as the predicted individual profiles well described the observed data as shown in Figure 2 for the predicted AUCs compared to the observed AUCs.

### Population PD model

A two-stage approach was used for the PD model development. Based on the physiological rationale, an indirect response model was built to describe the effect of Teverelix-TFA on suppression of testosterone, see Figure 1B. Plasma concentrations of teverelix were modelled to inhibit the production rate of testosterone. The parameters for the pharmacodynamic model are provided in Table 4 below.

| Parameter | Symbol (unit) | Estimate | RSE (%) | BSE | BSE RSE (%) |
|---|---|---|---|---|---|
| Maximum inhibition of production rate | IMAX | 0.953 | 0.4 | 79 | 7.3 |
| Teverelix concentration at 50% of maximum inhibition | IC50 (ng/mL) | 2.715 | 1.1 | 59 | 8.3 |
| Hill coefficient | H | 5.415 | 1 | 28 | 72.6 |
| Formation rate of testosterone | KFT (ng/mL*h) | 0.075 | 29.3 | 0.05 | FIXED |
| Fractional elimination rate of testosterone | KDEGT (1/h) | 0.023 | 85 | 30 | 15.9 |
| Residual Error | | | | | |
| Additive Error (ng/mL | ADD | 2.91E-06 | FIXED | | |
| Proportional Error | PROP | 0.434 | 3 | | |

| | | | | | |
|---|---|---|---|---|---|
| Table 4 PD parameters derived from the final population PD model BSE: between subject variation; RSE: relative standard error | | | | | |

Model qualification was conducted using diagnostic plots and posterior predictive checks to select a parsimonious model that is fit-for-purpose. Model diagnostic plots included basic diagnostic plots used in population modelling and individual predictions versus time. Numerical predictive check was performed by comparing observed and predicted castration rates in various studies. Successful clinical castration was defined as achieving plasma testosterone level <0.5 ng/mL within 28 days and maintain below this level for the duration of effect.

Various candidate dosage regimens were chosen for simulations using the population PK-PD model to select a suitable dosage regimen that meets the successful castration criteria following a review of PK-PD modelling results. Simulations were conducted using the individual pharmacokinetic/pharmacodynamic parameters (EBE) from all the subjects in modelling exercise. The simulated plasma testosterone concentrations were used to calculate the percent castration rate at various time points. Candidate dosage regimens that provide greater than or equal to 95% castration rates were selected as the recommended dosage regimen for future studies.

Basic diagnostic plots of pharmacokinetic/pharmacodynamic model showed no major bias and were acceptable. Individual predictions of both teverelix and testosterone matched with observed data. For instance, numerical predictive checks showed good agreement between observed and predicted castration outcome from various studies in all dose groups. A representative numerical predictive check from a study (called ANT-1111-02) is shown in table 5 and 6 with two different dosage regimes, where castration rate predicted by the model was within ~10% of the observed data for the higher dose group in the study.
Teverelix-TFA - LD: 120 mg IM; MD 120 mg SC Q6W

**Table 5: Base PK IDR with teverelix inhibition on testosterone:**

| Day | Castration rate (%) Observed in ANT-1111-02 | Castration rate (%) Predicted with the model |
|---|---|---|
| 28 | 67 (6/9) | 56 (5/9) |
| 42 | 56 (5/9) | 44 (4/9) |
| 168 | 38 (3/8) | 38 (3/8) |
| 196 | 38 (3/8) | 38 (3/8) |

Teverelix-TFA - LD: 180 mg IM; MD 180 mg SC Q6W

**Table 6: Base PK IDR with teverelix inhibition on testosterone:**

| Day | Castration rate (%) Observed in ANT-1111-02 | Castration rate (%) Predicted with the model |
|---|---|---|
| 28 | 98 (40/41) | 90 (37/41) |
| 42 | 80 (33/41) | 80 (33/41) |
| 168 | 62 (16/26) | 62 (16/26) |
| 196 | 55 (12/22) | 55 (12/22) |

The model was accordingly considered appropriate for applying for simulations to predict appropriate dosage regimen for future studies.

### Simulations

Castration levels are expected within 28 Days after start of treatment. The PK profile in the first 4 weeks of treatment is important to achieve and maintain castration levels and a loading dose may help in a fast onset of action.

Initial model simulations of average PK profiles substantiated the importance of an initial loading dose to achieve immediate castration levels. The IM administration has a faster absorption compared to the SC administration and a loading with a higher SC administration alone is not sufficient to achieve immediate castration levels. A combination of an IM and SC administration is therefore relevant to achieve immediate castration levels as a result of the IM administration and maintain the castration levels due to the slower second peak of the SC administration.

Based on these observations in the clinical trails various dosage regimens were simulated including
a. different loading doses: 180 to 480 mg SC alone, and 240 to 360 mg SC combined with 180 mg IM;
b. different maintenance doses: 240 to 900 mg SC; and
c. different dosage regimen: 6-weekly dosing (Q6W) and 8-weekly dosing (Q8W).

The simulated castration rates for the different dosing scenarios are shown in table 7, and the castration rates predicted using the model for the various candidate dosing scenarios are provided in table 8. Typical PK profiles of teverelix of four different dosage regimens are shown in Figure 3.

**Table 7. Simulation scenarios for various dosing strategies of loading and maintenance doses of teverelix-TFA**

| **Dosage Regimen** | **Loading Dose (LD)** | **Maintenance Dose (MD) Starting from week 4, SC injection** | **Frequency of administration for MD** |
|---|---|---|---|
| Scenario 1, Q6W | 360 mg SC | 240 mg/ 360 mg/480 mg/540 mg/630mg/720 mg/810 mg SC/900 mg | Q6W |
| Scenario 2, Q6W | 240 mg SC + 180 mg IM | 240 mg/ 360 mg/480 mg/540 mg/630mg/720 mg/810 mg SC/900 mg | Q6W |
| Scenario 3, Q6W | 360 mg SC + 180 mg IM | 240 mg/ 360 mg/480 mg/540 mg/630mg/720 mg/810 mg SC/900 mg | Q6W |
| Scenario 4, Q6W | 180 mg SC QD for 3 days | 240 mg/ 360 mg/480 mg/540 mg/630mg/720 mg/810 mg SC/900 mg | Q6W |
| Scenario 5, Q6W | 480 mg SC | 480 mg | Q6W |
| Scenario 6, Q6W | 360 mg SC | 240 mg/ 360 mg/480 mg/540 mg/630mg/720 mg/810 mg SC/900 mg | Q6W |
| Scenario 7, Q8W | 240 mg SC + 180 mg IM | 240 mg/ 360 mg/480 mg/540 mg/630mg/720 mg/810 mg SC/900 mg | Q8W |
| Scenario 8, Q8W | 360 mg SC + 180 mg IM | 240 mg/ 360 mg/480 mg/540 mg/630mg/720 mg/810 mg SC/900 mg | Q8W |
| Scenario 9, Q8W | 180 mg SC QD for 3 days | 240 mg/ 360 mg/480 mg/540 mg/630mg/720 mg/810 mg SC/900 mg | Q8W |
| Scenario 10, Q8W | 480 mg SC | 480 mg | Q8W |

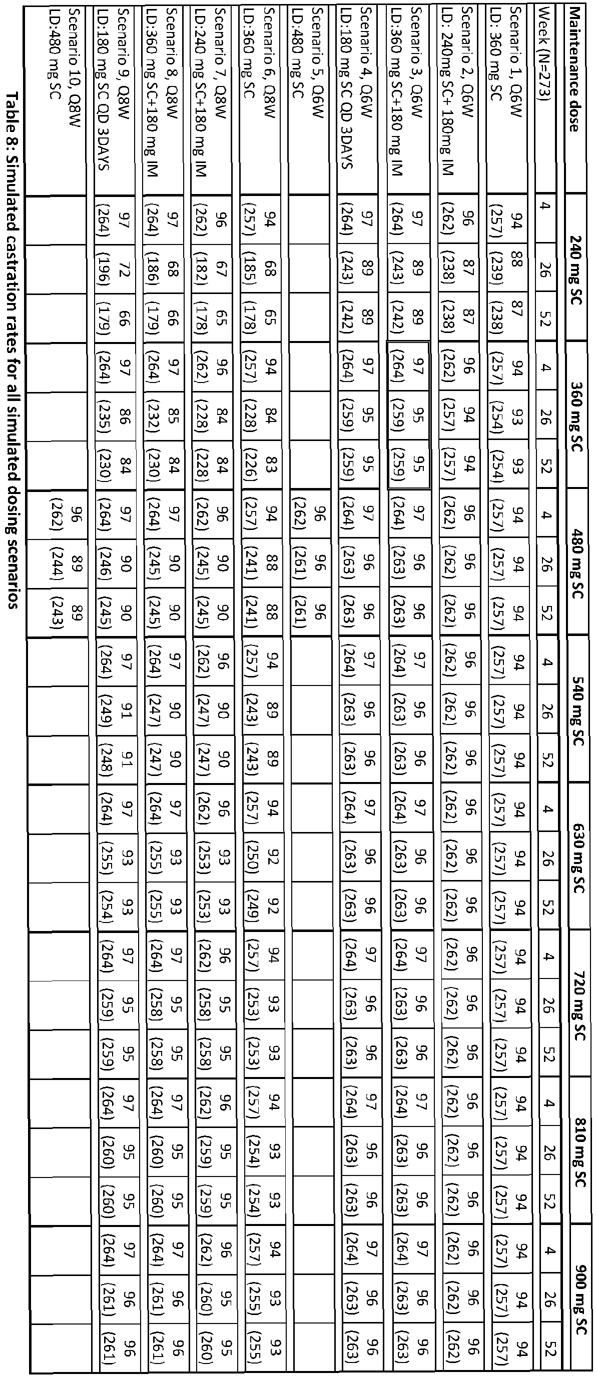

From table 8 it is clear that several different loading doses (LD) are capable of castrating the prostate cancer patients and maintain castration during the first 4 week dosing interval. However, most of said scenarios either require severely injections on consecutive days and/or relatively high maintenance doses.

For instance, loading with a 360 mg SC dose alone, a 360 mg IM loading dose alone, or lower combined loading doses of a teverelix-TFA with a dual SC+IM administration has proven not to sustain the teverelix plasma concentrations required to achieve the desired castration rate of ≥95% for the entire treatment period.

As an example can be mentioned that scenario 6 and 9 would not meet the practical criterion of being able to provide the loading dose in a single visit, as the patient needs to receive three subcutaneous injections every day (QD) in three days in order to obtain sufficient serum concentrations for achieving castration, and scenario 2 and 8 required much higher maintenance doses, respectively 480 mg SC and 720 mg SC in order to maintain a castration rate of ≥95%.

Thus, even though the differences between PK profiles following SC or IM or SC+IM loading dose scenarios become normalized over a period, the difference in PK profile in first four weeks is relevant for maintaining the target castration rate during a treatment period of at least 26 weeks, preferably at least 52 weeks.

As is evident from table 8, a loading dose of 360 mg SC and 180 mg IM with a maintenance dose of 360 mg of the teverelix-TFA administered subcutaneously is the lowest possible dose that can provide the desired castration rate during a treatment period of 52 weeks. The respective doses are highlighted with a box in table 8.

The first maintenance dose is administered on day 28 after the loading dose. Comparing scenario 3 and 8 having identical loading doses, but respectively following a Q6W and a Q8W dosing schedule dosages, it is clear than longer duration between the maintenance doses, i.e. eight-weeks cannot maintain the desired castration rate of ≥95% unless the patient receive at least 720 mg SC, i.e. twice the dose of the teverelix-TFA composition.

Thus, in order to reduce and minimize any dose-dependent adverse events and/or side effect, in particular the cardiovascular diseases it is preferred to use the lower maintenance dose of 360 mg SC, even though the patient has to receive an injection every six weeks, instead of every eight weeks.

While exemplary embodiments have been shown and described herein, it will be obvious to those skilled in the art that such embodiments are provided by way of example only. Numerous variations, changes, and substitutions will occur to those skilled in the art. It should be understood that various alternatives to the embodiments described herein may be employed. It is intended that the following claims define the scope of the disclosure and that composition within the scope of these claims and their equivalents be covered thereby

## Claims

1. A teverelix-TFA composition for use in the treatment of prostate cancer, wherein said composition is administered via a dosage regime comprising a loading dose and at least one maintenance dose, and wherein the loading dose comprises
- administering 360 mg of the teverelix-TFA composition subcutaneously,
- administering 180 mg of the teverelix-TFA composition intramuscularly, and
wherein the subcutaneously and intramuscularly administration are carried out substantially simultaneously.

2. A teverelix-TFA composition for use according to claim 1, wherein the loading dose of 360 mg teverelix-TFA subcutaneously and 180 mg teverelix-TFA intramuscularly is administered within a period of not more than 1 hour, even more preferred within a period of not more than 10 minutes, and even more preferred within a period of not more than 5 minutes.

3. A teverelix-TFA composition for use according to claim 1 or 2, wherein the maintenance dose comprises administering 360 mg of the teverelix-TFA composition subcutaneously.

4. A teverelix-TFA composition for use according to any of the preceding claims, wherein a first maintenance dose is administered 28 days after the loading dose.

5. A teverelix-TFA composition for use according to claim 4, wherein one or more second maintenance doses are administered every six weeks, starting from the day the first maintenance dose was administered.

6. A teverelix-TFA composition for use according to any of the preceding claims, wherein said dosage regime provide a castration rate of ≥95% during a treatment period of at least 26 weeks, preferably at least 52 weeks.

7. A teverelix-TFA composition for use according to any of the preceding claims, wherein the teverelix-TFA composition is a microcrystalline suspension.

8. A teverelix-TFA composition for use according to claim 7, wherein said microcrystalline suspension comprises from 2.0 mol to 2.8 mol TFA for each mol teverelix in said composition.

9. A teverelix-TFA composition for use according to any of the preceding claims, wherein the dosage regime provide a blood plasma testosterone concentration in a male subject of 0.5 ng/mL or less at least 72 hours after the administering of the loading dose, and wherein said blood plasma testosterone concentration is measured by a liquid chromatography/mass spectrometry (LC-MS) assay.

10. A teverelix-TFA composition for use according to claim 9, wherein a blood plasma testosterone concentration of less than about 0.5 ng/mL is maintain for a treatment period of at least 26 weeks, preferably at least 52 weeks.

11. A teverelix-TFA composition for use according to any of the preceding claims, wherein the dosage regime comprises administering an additional pharmaceutical substance, preferably selected from the group comprising antineoplastics, adrenergic receptor antagonists and 5α-reductase inhibitors.

12. A pharmaceutical formulation comprising the composition according to any of the claims 1 - 11 for use in the treatment of prostate cancer.

13. A pharmaceutical formulation for use according to claim 12, wherein the formulation further comprises an isotonic agent, preferably selected from mannitol or polyhedric alcohol, e.g. a sugar alcohol and/or an a pharmaceutically acceptable excipients.

14. A kit comprising the composition according to any of the claims 1 - 11 or a pharmaceutical formulation according to any of the claims 12 - 13 for use in the treatment of prostate cancer, wherein said kit further least one syringe for administration of the teverelix-TFA composition.
